# EUROPÄISCHE PATENTSCHRIFT

(11) **EP 1 730 315 B1**
(45) Veröffentlichungstag und Bekanntmachung des Hinweises auf die Patenterteilung: **21.10.2009**
(21) Anmeldenummer: 05736111.5
(22) Anmeldetag: 29.03.2005
(51) Int. Cl.: C12Q 1/68

(54) **POLYMORPHISMEN IM NOD2/CARD15 GEN**
POLYMORPHISMS IN NOD2/CARD15 GENE
POLYMORPHISMES DANS LE GENE NOD2/CARD15

(30) Priorität: 27.03.2004 DE 102004015143
(43) Veröffentlichungstag der Anmeldung: 13.12.2006
(73) Patentinhaber: Klein, Hanns-Georg, 82152 Martinsried (DE)
(72) Erfinder: Klein, Hanns-Georg, 82152 Martinsried (DE)
(74) Vertreter: Arth, Hans-Lothar
(86) Internationale Anmeldenummer: PCT/DE2005/000550
(87) Internationale Veröffentlichungsnummer: WO 2005/095640

(56) Entgegenhaltungen:
- WO-A-02/44426
- WO-A-03/060468
- US-A1- 2004 053 263
- LESAGE SUZANNE ET AL: "CARD15/NOD2 mutational analysis and genotype-phenotype correlation in 612 patients with inflammatory bowel disease." AMERICAN JOURNAL OF HUMAN GENETICS. APR 2002, Bd. 70, Nr. 4, April 2002 (2002-04), Seiten 845-857, XP002340892 ISSN: 0002-9297
- HAMPE J ET AL: "Association of NOD2 (CARD 15) genotype with clinical course of Crohn's disease: a cohort study" LANCET THE, LANCET LIMITED. LONDON, GB, Bd. 359, Nr. 9318, 11. Mai 2002 (2002-05-11), Seiten 1661-1665, XP004790813 ISSN: 0140-6736
- RAHMAN P ET AL: "CARD15: a pleiotropic autoimmune gene that confers susceptibility to psoriatic arthritis." AMERICAN JOURNAL OF HUMAN GENETICS. SEP 2003, Bd. 73, Nr. 3, September 2003 (2003-09), Seiten 677-681, XP002340893 ISSN: 0002-9297
- HUGOT JEAN-PIERRE ET AL: "Association of NOD2 leucine-rich repeat variants with susceptibility to Crohn's disease" NATURE, MACMILLAN JOURNALS LTD. LONDON, GB, Bd. 411, Nr. 6837, 2001, Seiten 599-603, XP002177308 ISSN: 0028-0836 in der Anmeldung erwähnt
- OGURA YASUNORI ET AL: "A frameshift mutation in NOD2 associated with susceptibility to Crohn's disease" NATURE, MACMILLAN JOURNALS LTD. LONDON, GB, Bd. 411, Nr. 6837, 2001, Seiten 603-606, XP002177309 ISSN: 0028-0836 in der Anmeldung erwähnt
- HOLLER ERNST ET AL: "Both donor and recipient NOD2/CARD15 mutations associate with transplant-related mortality and GvHD following allogeneic stem cell transplantation" BLOOD, Bd. 104, Nr. 3, 1. August 2004 (2004-08-01), Seiten 889-894, XP002340894 ISSN: 0006-4971
- SCHUERMANN M. ET AL: 'CARD15 GENE MUTATIONS IN SARCOIDOSIS' EUROPEAN RESPIRATORY JOURNAL Bd. 22, Nr. 5, 01 November 2003, Seiten 748 - 754, XP009035144 ISSN: 0903-1936

## Beschreibung

Die vorliegende Erfindung betrifft Verfahren sowie in diesen Verfahren verwendete Nucleotidsequenzen zur Vorhersage und/oder Diagnose von Krankheiten, welche mit mindestens einem der Polymorphismen Nod2-SNP8, Nod2-SNP12, Nod2-SNP13 im NOD2/CARD15 Gen in Verbindung stehen und insbesondere die Auswahl geeigneter Spender-Empfänger-Paare für Transplantationen aufgrund der nachgewiesenen Polymorphismen Nod2-SNP8 (8), Nod2-SNP12 (12), Nod2-SNP13 (13) im NOD2/CARD15 Gen.

Die Einzelnucleotidpolymorphismen (SNP: single nucleotide polymorphism) 8, 12, 13 im NOD2/CARD15 Gen werden für das Auftreten der Crohnschen Krankheit verantwortlich gemacht (Hugot JP, Chamaillard M, Zouali H, Nature 2001, 411, 599-603; Ogura Y, Bonen DK, Inohara N Nature 2001, 411, 603-606). Bei der Crohnschen Krankheit handelt es sich um eine zwar seltene, aber verheerende chronisch wiederkehrende Entzündung des Verdauungstraktes, die vor allem Dünn- und Dickdarm befällt. Dabei kommt es zu einer Verdickung der Darmwand und zu Geschwürbildungen.

WO 02/44426 A beschreibt eine Möglichkeit zur Vorhersage und/oder Diagnose von Patienten mit einem Risikofaktor für die Crohnsche Erkrankung durch Detektion der Anwesenheit zumindest eines Polymorphismus im NOD2/CARD15 Gen. WO 03/060468 A offenbart die Möglichkeit zur Bestimmung der Wahrscheinlichkeit einer Abstoßungsreaktion bei Transplantationen durch Detektion der Anwesenheit zumindest eines Polymorphismus in dem humanen Mif Promotorgen. Schürmann et al., European Respiratory Journal 2003, 22(5), 748-754 offenbart die Prognose und/oder Diagnose von Sarkoidose durch Detektion der Anwesenheit eines Polymorphismus NOD2-SNP12 im NOD2/CARD15 Gen. Keines der vorgenannten Dokumente offenbart jedoch alleine oder auch in Kombination miteinander die vorliegende Erfindung.

Aufgabe der vorliegenden Erfindung ist es nun, Verfahren zur Vorhersage und Diagnose von Krankheiten bereitzustellen, welche durch die MutationenNarianten Nod2-SNP8, Nod2-SNP12, Nod2-SNP13 im NOD2/CARD15 Gen ausgelöst oder initiiert werden. Insbesondere besteht die Aufgabe darin, Verfahren zur Vorhersage der Wahrscheinlichkeit des Auftretens von Transplantat-gegen-Wirt-Reaktionen und anderer nach Transplantationen auftretender Erkrankungen bereitzustellen, welche zur Auswahl geeigneter Spender-Empfänger-Paare dienen.

Diese Aufgabe wird durch die Bereitstellung von Verfahren gemäß Anspruch 1 gelöst. Weitere vorteilhafte Ausgestaltungen, Aspekte und Details der Erfindung ergeben sich aus den abhängigen Ansprüchen, der Beschreibung, den Beispielen und den Figuren.

Die vorliegende Erfindung betrifft Verfahren zur Vorhersage und/oder Diagnose von mit mindestens einem der Polymorphismen Nod2-SNP8, Nod2-SNP12, Nod2-SNP13 im NOD2/CARD15 Gen assoziierten Krankheiten durch Nachweis mindestens eines der Polymorphismen Nod2-SNPB, Nod2-SNP12, Nod2-SNP13 im NOD2/CARD15 Gen.

Anders ausgedrückt betrifft die vorliegende Erfindung Verfahren zum Nachweis mindestens eines der Polymorphismen Nod2-SNP8, Nod2-SNP12, Nod2-SNP13 im NOD2/CARD15 Gen zur Vorhersage und/oder Diagnose von mit diesem Gendefekt bzw. einer Mutation bzw. einer Genvarianten assoziierten Krankheiten.

Das NOD2/CARD15 Gen hat folgende Gene Bank Accession Number: AC007728 und AQ534686. Die Nucleotidsequenz ist unter den angegebenen Accession Nummern aus der NCBI-Datenbank unter http://www.ncbi.nlm.nih.gov/ erhältlich.

Das NOD2-Gen ist 3123 Nukleotide lang (1040 Aminosäuren, Gene Bank Accession number NM_022162), (NOD: Nucleotide Oligomerisation Domain) und befindet sich in der perizentromerischen Region des Chromosoms 16 (16p12-q21). Die Nomenklatur des Gens NOD2 wurde inzwischen geändert zu CARD15 (CARD: Caspase Activating Recruitment Domain). Caspasen spielen eine wichtige Rolle bei der Apoptose. Neben der CARD Domäne besitzt NOD2/CARD15 eine ATB Bindungsdomäne. NOD2/CARD15 dient als intrazellulärer Rezeptor für bakterielle Produkte und transduziert das Signal zur Aktivierung von NFkappaB (NF-κB).

In dem NOD2/CARD15 Gen können unter anderem Einzelnucleotidpolymorphismen (SNPs) Nod2-SNP8, Nod2-SNP12, Nod2-SNP13 auftreten. SNPs entstehen durch einen Basenaustausch oder Insertion einer zusätzlichen Base in die DNA-Sequenz.

SNP8 (SNP Datenbank http://www.ncbi.nlm.nih.gov/SNP/index.html, Accession Number ss2978536) bezeichnet einen Polymorphismus im NOD2/CARD15 Gen, der durch den Austauschs des Nucleotids Position 2209 (NM_022162) C → T entsteht. Resultierend findet im Protein der Austausch von R702W statt. SNP8 befindet sich im Chromosom 16 an der Chromosomenposition 50523959 (NOD2/CARD15 Gen - Exon 5). Hugot JP et al., Association of NOD2 leucine-rich repeat variants with susceptibility to Crohn's disease. Nature 2001, 411, 599-603.

SNP12 (SNP Datenbank, Accession Number ss2978537) bezeichnet einen Polymorphismus im NOD2/CARD15 Gen, der durch den Austauschs des Nucleotids Position 2827 (NM_022162) G → C entsteht. Resultierend findet im Protein der Austausch von G908R statt. SNP12 befindet sich im Chromosom 16 an der Chromosomenposition 50534573 (NOD2/CARD15 Gen- Exon 9). Hugot JP et al., Association of NOD2 leucine-rich repeat variants with susceptibility to Crohn's disease. Nature 2001, 411, 599-603

SNP13 (SNP Datenbank, Accession Number ss2978539) bezeichnet einen Polymorphismus im NOD2/CARD15 Gen, der durch 1 Base Insertion des Nucleotids C am Nucleotid Position 3124 (NM_022162). SNP13 befindet sich im Chromosom 16 an der Chromosomenposition 50541811^50541812. Durch die Insertion resultiert ein Frameshift, der ein verkürztes NOD2 mit 1007 Aminosäuren bedingt (Ogura et al., Nature 2001, 411, 603-606).

Durch die oben genannten Polymorphismen Nod2-SNP8, Nod2-SNP12, Nod2-SNP13 entstehen folgende 7 Mutanten / Varianten, nämlich
Mutante 1: Mutation durch SNP8,
Mutante 2: Mutation durch SNP12,
Mutante 3: Insertion durch SNP13,
Mutante 4: Mutationen durch SNP8 und 12,
Mutante 5: Mutation und Insertion durch SNP8 und 13,
Mutante 6: Mutation und Insertion durch SNP12 und 13,
Mutante 7: Mutationen und Insertion durch SNP8, 12 und 13.

Überraschenderweise konnte nachgewiesen werden, dass bereits eine der vorgenannten Mutationen ausreicht um diverse Krankheiten auszulösen. Die vorgenannten Mutationen stehen beispielsweise mit Abstoßungsreaktionen in der Transplantationsmedizin, Graft-versus-Host-Erkrankungen, Host-versus-Graft-Erkrankungen und Bronchiolitis Obliterans in Verbindung.

Zu den Abstoßungsreaktionen bei Transplantationen zählen insbesondere immunologische Reaktionen des Empfängers gegen das Spenderorgan als auch Transplantat gegen Wirt Reaktionen (GvHD: Graft versus Host Disease). Derartige Folgen treten relativ spät nach einer Transplantation auf und können zu lebensbedrohlichen Komplikation führen.

Diese Problematik soll am Beispiel der Blutstammzelltransplantation, herkömmlich als Knochenmarktransplantation bekannt, verdeutlicht werden. Beispiele für Transplantationen sind insbesondere Rückenmark-, Knochenmark-, Stammzell- und Blutstammzelltransplantationen sowie Transplantationen solider Organe wie beispielsweise Herz, Lunge, Leber, Niere, Bauchspeicheldrüse, Haut, hormonproduzierende Drüsen sowie Keimdrüsen.

Die Blutstammzelltransplantation kann zur Therapie unterschiedlicher Störungen der Blutbildung sowie des Immunsystems eingesetzt werden, welche sich beispielsweise nach Chemotherapien oder anderer die Blutbildung zerstörender Therapien ergeben können. Die Störungen des Immunsystems oder bei der Blutbildung können aber auch angeborener oder erworbener Natur sein. Probleme bei der Blutstammzelltransplantation können insbesondere dann auftreten, wenn der Spender genetisch nicht identisch ist (allogener Spender), da in diesem Fall eine doppelte immunologische Barriere überwunden werden muss. Denn zu der möglichen Abstoßung der transplantierten Zellen durch das Immunsystem des Empfängers (HvG-Reaktion) kommt auch noch die Möglichkeit des Auftretens immunologischer Reaktionen des Transplantats gegen den Empfänger (GvH-Reaktionen) hinzu.

Ein Faktor, der die Wahrscheinlichkeit von Abstoßungsreaktionen wesentlich mitbestimmt, ist die Übereinstimmung der HLA (Human Leukocyte Antigens)-Haplotypen zwischen Spender und Empfänger.

Die vorliegende Erfindung offenbart unter anderem nun einen weiteren wesentlichen Faktor für die Wahrscheinlichkeit des Auftretens von Abstoßungsreaktionen, nämlich das Vorliegen mindestens eines der Polymorphismen SNP8, SNP12 oder SNP13 im NOD2/CARD15 Gen. Insbesondere nachteilig wirkt sich das Vorliegen mindestens eines dieser Polymorphismen bei dem Spender und dem Empfänger aus.

Die Graft-versus-Host-Reaktion (GvHR), welche durch T-Lymphozyten, die im Transplantat des Spenders enthalten sind, ausgelöst wird, kann zu einer GvHD führen. Bei der GvHD wird eine akute und eine chronische Form unterschieden. Per definitionem tritt die akute GvHD innerhalb der ersten 100 Tage nach der Transplantation und bei bis zu 50% der Transplantierten auf. Die chronische GvHD tritt nach dem 100. Tag bei etwa 30-50% der allogen Transplantierten auf. Entsprechend der Symptomatik und klinische n Laborwerte kann die GvHD in vier Stadien (GvHD-I, GvHD-II, GvHD-III, GvHD-IV) mit unterschiedlichen Prognosen eingeteilt werden.

Eine weitere schwere und oft tödlich verlaufende Folgeerkrankung nach Transplantationen ist Sepsis, welche beispielsweise durch mit dem Transplantat übertragene Bakterien ausgelöst werden kann. Ca. 100.000 Menschen erkranken in Deutschland jährlich an einer Sepsis, 40.000 von ihnen sterben daran. Bei einer Sepsis überschwemmen Keine den gesamten Organismus und vergiften das Blut, wodurch Niere, Leber und Lunge versagen können. Sepsis ist eine Killerkrankheit, welche von Bakterien, Pilzen oder Viren hervorgerufen wird und jeden plötzlich treffen kann.

Insbesondere vorteilhaft ist die Anwendung der erfindungsgemäßen Verfahren für die Prätransplantationsdiagnostik. Die erfindungsgemäßen Verfahren ermöglichen die Vorhersage von Abstoßungsreaktionen des Empfängers gegenüber dem Spenderorgan sowie Transplantat gegen Wirt Reaktionen (GvHD) und dienen somit dazu, für einen bestimmten Empfänger ein geeignetes Spenderorgan auszuwählen. Insbesondere treten dann Abstoßungsreaktionen auf, wenn sowohl der Empfänger als auch der Spender eine der oben beschriebenen Mutationen 1 - 7 aufweisen, wie weiter unten noch ausführlich ausgeführt wird.

Die erfindungsgemäßen Verfahren umfassen das Bereitstellen einer Probe, welche das Gen NOD2/CARD15 enthält und die anschließende Analyse des Gens NOD2/CARD15 auf das Vorliegen mindestens einer der oben beschriebenen Mutationen 1 - 7. Vorzugsweise wird für die Untersuchung genomische DNA oder cDNA oder RNA verwendet.

Als Probe können Blut, Speichel / Mundschleimhautzellen, Knochenmark, Urinsediment, Punktatflüssigkeit, Zell- sowie Gewebeproben verwendet werden, wobei Blutproben bevorzugt sind.

Bezüglich der Prätransplantationsdiagnostik ist es zudem bevorzugt, eine Probe des Empfängers sowie separat davon auch eine Probe des Spenders zu untersuchen. Insbesondere bevorzugt ist somit ein Diagnoseverfahren, welches zur Abschätzung der Wahrscheinlichkeit des Auftretens von Transplantat gegen Wirt Reaktionen dient, umfassend die folgenden Schritte:
a) Bereitstellung einer Probe des Spenders enthaltend das Gen NOD2/CARD15 sowie eine Probe des Empfängers enthaltend das Gen NOD2/CARD15,
b) Detektion der beiden Proben auf das Vorliegen eines oder mehrerer der Polymorphismen Nod2-SNP8, Nod2-SNP12, Nod2-SNP13.

Die nachfolgende Auswahl von passenden Spender-Empfänger-Paaren erfolgt nach dem Grundsatz der Minimierung des Risikos bezüglich der Wahrscheinlichkeit des Auftretens von Transplantat gegen Wirt Reaktionen. Weist der Empfänger bereits mehr als einen Polymorphismus auf, d.h. weist der

Empfänger eine der Mutationen Nr. 4, 5, 6 oder 7 auf, dann sollte der Spender keinen einzigen Polymorphismus, d.h. keine der Mutationen Nr. 1 - 7 besitzen. Ein besonderes hohes Risiko von GvHD besteht, wenn sowohl Spender als auch Empfänger einen Polymorphismus aufweisen und ein nochmals gesteigertes Risiko, wenn Spender und Empfänger insgesamt mehr als zwei Polymorphismen aufweisen, z.B. beim Spender die Mutation 5 und beim Empfän ger die Mutation 1, oder beim Spender die Mutationen 4 und beim Empfänger die Mutation 6.

Das vorgenannte Verfahren umfasst in einer bevorzugten Ausführungsform die Schritte:
a) Bereitstellung einer Probe des Spenders sowie des Empfängers enthaltend das Gen NOD2/CARD15,
b) Isolation der DNA und/oder RNA aus beiden Proben,
c) Durchführung getrennter Polymerasekettenreaktionen mit für das Gen NOD2/CARD15 spezifischen Primern für isolierte DNA und/oder RNA des Spenders als auch für die isolierte DNA und/oder RNA des Empfängers,
d) Untersuchung des Gens NOD2/CARD15 des Spenders als auch des Gens NOD2/CARD15 des Empfängers auf das Vorliegen mindestens eines der Polymorphismen Nod2-SNP8, Nod2-SNP12, Nod2-SNP13.

Eine bevorzugte Ausführungsform der vorliegenden Erfin dung betrifft ein Verfahren zur Abschätzung bzw. Vorhersage von Abwehrreaktionen beim Empfänger nach erfolgter Transplantation von Zellen oder eines Organs. Bei diesem Verfahren wird eine Probe des Empfängers und eine Probe des Spenders bereitgestellt. Der Begriff "Probe" ist oben definiert und bezeichnet insbesondere eine Blutprobe. Die Probe des Spenders als auch die Probe des Empfängers werden auf das Vorliegen der Polymorphismen SNP8, SNP12, SNP13 untersucht. Vorzugsweise erfolgt diese Untersuchung durch die Isolation von DNA und/oder RNA, vorzugsweise DNA aus beiden Proben. Die aus der Probe des Spenders isolierte DNA und/oder RNA wird mittels PCR vervielfältigt und anschließend zum Nachweis der Polymorphismen mit Oligonucleotiden versetzt, welche eine zum Polymorhismus SNP8 oder SNP12 oder SNP13 komplementäre Sequenz aufweisen und zur Hybridisierung mit dem Bereich des Gen s NOD2/CARD15 befähigt sind, der den Polymorphismus Nod2-SNP8 bzw. Nod2-SNP12 bzw. Nod2-SNP13 enthält. Die Oligonucleotide weisen bevorzugt zwischen 10 und 50 Nucleotidbausteine (Nucleobasen) auf. Eingesetzt werden drei Arten von Oligonucleotiden, nämlich solche mit einer komplementären Sequenz zu dem Abschnitt des NOD2/CARD15 Gens, der den SNP8 Polymorphismus enthält sowie solche mit einer komplementären Sequenz zum dem Abschnitt des NOD2/CARD15 Gens, der den SNP12 Polymorphismus enthält und solche mit einer komplementären Sequenz zum dem Abschnitt des NOD2/CARD15 Gens, der den SNP13 Polymorphismus enthält. Das Auftreten von Hybridisierung belegt das Vorhandensein eines Polymorphismus. Die Art der hybridisierenden Oligonucleotide gibt Auskunft darüber, welcher Polymorphismus vorliegt und ob mehrere Polymorphismen vorliegen. Die PCR wird mit für das Gen NOD2/CARD15 spezifischen Primern durchgeführt.

Die Probe des Empfängers wird zur Vervielfältigung der darin enthaltenen DNA und/oder RNA auch einer PCR unterzogen und zum Nachweis von Polymorphismen mit den gleichen Oligonucleotiden versetzt.

Das erfindungsgemäße Verfahren gibt nun Auskunft darüber, ob beim Spender oder beim Empfänger oder bei Spender und Empfänger Polymorphismen vorliegen. Ferner kann bestimmt werden, welche und wie viele der drei Polymorphismen vorliegen. Je nach Ergebnis kann die Wahrscheinlichkeit von Abwehrreaktionen beim Transplantatempfänger abgeschätzt werden, wodurch sich die Möglichkeit ergibt, zueinander passende. Spender-Empfänger-Paare auszuwählen und die Wahrscheinlichkeit von Folgeerkrankungen aufgrund der Transplantation zu minimieren.

Unter Folgeerkrankungen oder Abwehrreaktionen werden Abstoßungsreaktionen nach Transplantationen, Graft-versus-Host-Erkrankungen, Host-versus-Graft-Erkrankungen, und/oder Bronchiolitis Obliterans verstanden,

In experimentellen Studien konnte gezeigt werden, dass das Vorliegen einer der Polymorphismen mit dem gehäuften Auftreten von Abstoßungsreaktionen nach Transplantationen, Graft-versus-Host-Erkrankungen, Host-versus-Graft-Erkrankungen und Bronchiolitis obliterans korreliert. Die erfindungsgemäßen Verfahren führen zu Ergebnissen, welche eine Aussage über die Wahrscheinlichkeit des Auftretens der vorgenannten Erkrankungen zulassen. Insbesondere der Wahrscheinlichkeit des Auftretens einer oder mehrerer der vorgenannten Erkrankungen beim Empfänger nach Transplantationen.

Wie oben ausgeführt, erfolgt nach dem Bereitstellen einer Probe, welche das Gen NOD2/CARD15 enthält, die Untersuchung des Gens bezüglich des Vorliegens mindestens einer der oben genannten Mutationen 1 - 7 bzw. mindestens eines der Polymorphismen 8, 12, 13. Diese Untersuchung dann durch verschiedene Methoden erfolgen, welche bevorzugt die Isolation der DNA aus der Probe mit nachfolgender Durchführung einer PCR (Polymerase Chain Reaction) mit für das Gen NOD2/CARD15 spezifischen Primern umfassen.

Als Primer können beispielsweise die in THE LANCET 2002, 359, 1661-1665 beschriebenen eingesetzt werden.

Die durch PCR erhaltenen Nucleotide können dann unter Verwendung diverser Methoden auf das Vorliegen der Mutationen untersucht werden. Zu diesen Methoden zählen beispielsweise: Primerextensionsverfahren, mutationsspezifische Hybridisierung, ARMS-Technologien, Array-Technologien, Chip-Technologien, DNA-Sequenzanalyse, Restriktionsanalyse (RFLP), Einzelstrang-Konformations-Polymorphismus (SSCP), denaturierende Gradienten-Gelelektrophorese, Gelelektrophorese mit Temperaturgradient, denaturierende HPLC, elektrochemische Nachweisverfahren. Insbesondere bevorzugt ist die Messung von Hybridisierungssignalen von markierten Hybridisierungssequenzen.

### Kurz Beschreiung der Methoden

Ein Verfahren zum Nachweis bekannter SNPs oder Mutationen, welches in der Routinediagnostik zum Einsatz kommt, ist die allelspezifische Hybridisierung von fluoreszenzmarkierten DNA-Sonden (allelspezifische Oligonucleotidhybridisierung oder **ASO**). Bei dieser Technik wird der PCR eine Wildtyp- und eine mutationsspezifische Sonde zugesetzt, deren Fluoreszenz erst sichtbar wird, nachdem die zuvor spezifisch gebundene Sonde durch die DNA-Polymerase im Zuge der Strangelongation abgebaut wurde (sog. Exonuklease-Assay). Je nachdem, ob Wildtyp oder SNP/Mutation vorliegt, erhält man unterschiedliche Farbsignale, welche nach Anregung durch Laserlicht in einer Photozelle detektiert werden. Der Vorteil dieses Verfahrens liegt in der Teilautomatisierbarkeit, wodurch größere Probenzahlen schneller bearbeitet werden können.

Ein neueres, in Schweden entwickeltes Verfahren zum Nachweis bekannter Mutationen oder SNPs ist das **Pyrosequencing.** Ähnlich der klassischen DNA-Sequenzanalyse mit dem Sanger-Verfahren (s.u.) kann mittels Pyrosequencing die DNA-Sequenz direkt analysiert werden. Allerdings beruht die Reaktion nicht auf der Detektion von Fluoreszenzsignalen von eingebauten Stop-Nukleotiden, sondern auf einer messbaren Freisetzung von Licht, wenn das jeweils passende Nukleotid bei der Strangelongation in die DNA eingebaut wird. Insbesondere werden zum Nachweis des Vorliegens einer Mutation Hybridisierungssequenzen, d.h. Oligonucleotide mit komplementären Sequenzen eingesetzt, welche vorzugsweise Detektionsmarker tragen.

Eine weitere Methode zur Analyse bekannter Polymorphismen oder SNPs ist der Nachweis über sequenzspezifische Sonden mittels **LightCycler.** Dabei wird zu einem DNA-Templat ein spezieller Reaktionsmix gegeben, der u.a. Primer und spezifische Sonden enthält. Für die Detektion eines SNP wird jeweils ein Sondenpaar benötigt, das aus einer Anker- und einer Sensor-Sonde besteht. Beide Sonden binden in unmittelbarer Nachbarschaft zueinander an die zu analysierende Sequenz. Nach Exzitation des Fluoresceinmoleküls an der Ankersonde kommt es zum Energietransfer auf das LC-Red-Molekül an der Sensorsonde (Fluoreszenz-Resonanz-Transfer, FRET-Assay). Das von der Sensorsonde emittierte Licht wird gemessen und steht dafür, dass die Sonden korrekt an die Zielsequenz gebunden sind. Die Ermittlung des Genotyps erfolgt über eine Schmelzkurvenanalyse.

Ein Verfahren, welches sich besonders zum simultanen Nachweis mehrerer bekannter Mutationen (sog. Multiplexanalyse) eignet, ist der **Oligonukleotid-Ligation-Assay (OLA).** Der OLA kommt vorwiegend in Stufe II (Analyse auf die 31 häufigsten Mutationen) der CFTR-Diagnostik zum Einsatz. In einer Multiplex-PCR-Reaktion werden 15 Zielregionen des CFTR-Gens amplifiziert. Die PCR-Produkte aus der Multiplex-PCR dienen als Ausgangsmoleküle für eine Ligationsreaktion, die durch Zugabe eines OLA-Reagenzes gestartet wird. Das OLA-Reagenz enthält Oligonukleotide, welche teilweise fluoreszenzmarkiert sind sowie eine DNA-Ligase. Bei der Reaktion werden 3 Sondentypen eingesetzt: a) eine gemeinsame ("common") Sonde, die am 3'-Ende mit einem Floureszenzfarbstoff (blau, grün oder gelb) markiert ist, b) 29 wildtypspezifische Sonden und c) 31 mutationsspezifische Sonden. Die gemeinsame Sonde bindet an die Zielsequenz unabhängig davon, ob eine Wildtyp- oder mutante Sequenz vorliegt. Die Wildtyp- und Mutationssonden unterscheiden sich nur durch ein Nukleotid am 3'-Ende. Am 5'-Ende von Wildtyp- und Mutationssonde hängt jeweils ein sog. PEO-Molekül, dessen Länge spezifisch für jede nachzuweisende Zielsequenz ist. Die Wildtyp- und Mutationssonden konkurrieren um die Bindungsstelle an die Zielsequenz, wobei nur die Sonde bindet, die exakt mit der Zielsequenz komplementär ist. Liegt keine der 31 zu analysierenden Mutationen vor, binden nur die Wildtypsonden. Ist eine Mutation homozygot vorhanden, bindet nur die Mutationssonde. Bei Heterozygotie binden sowohl Wildtyp- wie auch Mutationsonde an die Zielsequenz. Die DNA-Ligase verknüpft die gemeinsame und Wildtyp- oder Mutationssonde nach Hybridisierung an die Zielsequenz. Jedes Ligationsprodukt kann nun über seine spezifische Länge und die unterschiedliche Fluoreszenzmarkierung der gemeinsamen Sonde kapillarelektrophoretisch nachgewiesen werden.

Somit bezieht sich die vorliegende Erfindung auch auf Oligonucleotide, welche aus mindestens 10 Nucleotidbausteinen bestehen, eine zur Mutation SNP8 und/oder SNP12 und/oder SNP13 komplementäre Sequenz aufweisen und zur Hybridisierung mit dem Bereich des Gens NOD2/CARD15 befähigt sind, der den Polymorphismus Nod2-SNP8 und/oder Nod2-SNP12 und/oder Nod2-SNP13 enthält.

Beziehungsweise betrifft die vorliegende Erfindung Oligonucleotide bestehend aus mindestens 10 Nucleotiden, wobei die Oligonucleotide eine zum NOD2/CARD15 Gen komplementäre Sequenz haben und die komplementären Nucleotide zur Mutation SNP8 und/oder SNP12 und/oder die Nucleotidinsertion SNP13 enthalten.

Derartige Oligonucleotide tragen bevorzugt Detektionsmarker zur spektroskopischen Analyse bzw. zur Fluoreszenz- oder Chemilumineszenzbasierten Analyse. Ferner bestehen die Oligonucleotide bevorzugt aus 15 - 50 Nucleotidbausteinen und insbesondere bevorzugt aus 18 - 22 Nucleotidbausteinen.

Gegenstand der vorliegenden Erfindung ist ferner eine Vorrichtung für die Diagnose der Polymorphismen bzw. Mutationen des NOD2/CARD15 Gens. Bei dieser Vorrichtung handelt es sich bevorzugt um einen Diagnosechip oder Mikrochip, der mindestens eines der vorgenannten Oligonukleotide enthält. Bei dieser Mikroarray- oder Mikrochip-Technolgie werden auf speziellen Trägern, die zumeist aus Glas oder Nylon bestehen, in hoher Dichte tausende verschiedener Gene in Form von cDNA Fragmenten oder genspezifischen Oligonucleotiden mit Hilfe von Robotern aufgetragen (= DNA-Chips).

Anstelle des Einsatzes von DNA-Proben können auch RNA-Proben zum Nachweis der Polymorphismen eingesetzt werden. Die RNA-basierten Verfahren benötigen jedoch Proben von Zellen, welche auch das NOD2/CARD15 Gen exprimieren.

### Figurenbeschreibung

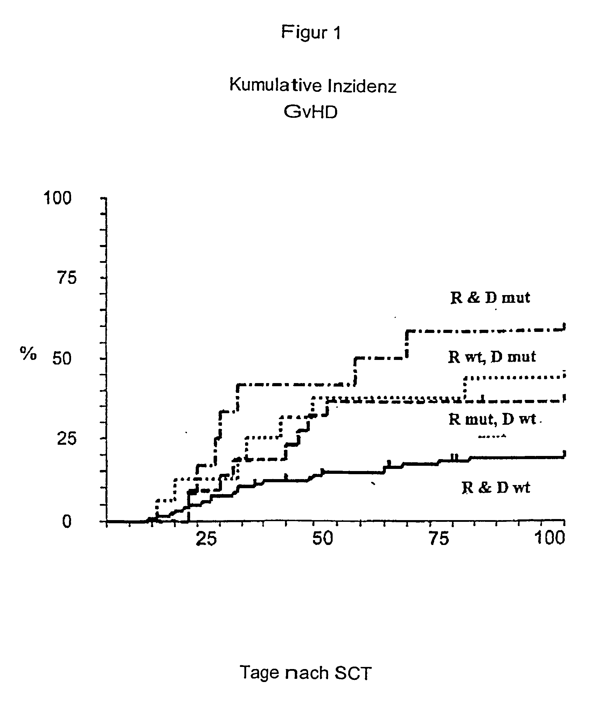

- Figur 1: zeigt die kumulative Inzidenz von schwerer akuten GvHD (Grad III/OV) in Bezug zu NOD2/CARD15 SNPs: R= Empfänger, D= Spender; R&D wt = nicht-mutantes Wildtyp SNPs in R und D (n=119); R mut, D mut = mutante SNPs nur in Empfänger (n = 22); R wt, D mut = mutantes SNPs nur in Spendern (n=16), R & D mut = mutantes SNPs in Spender und Empfänger (n=12). Die Unterscheide waren hoch signifikant: p 0,001 für die ganzen Gruppen; p 0,02 für R mut D wt; p 0,004 für R wt/Dmut und p 0,001 für R & D mut wenn es mit nicht mutantem R & D verglichen wurde.

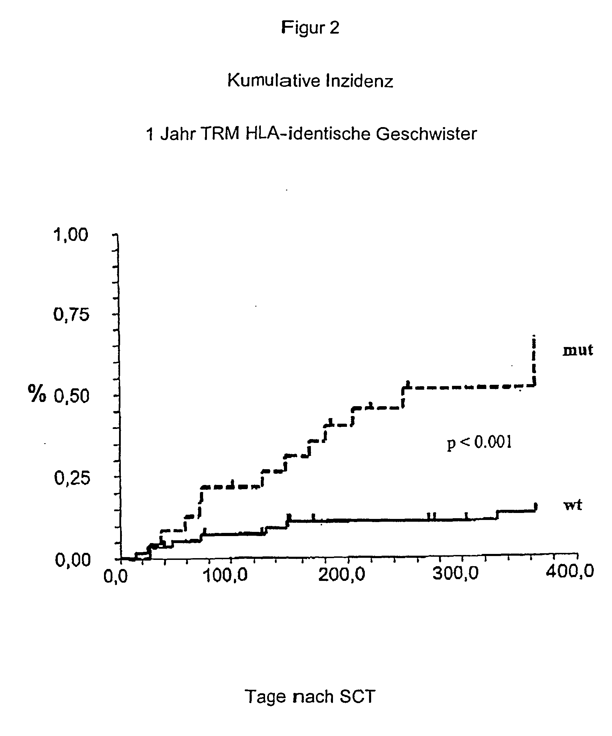

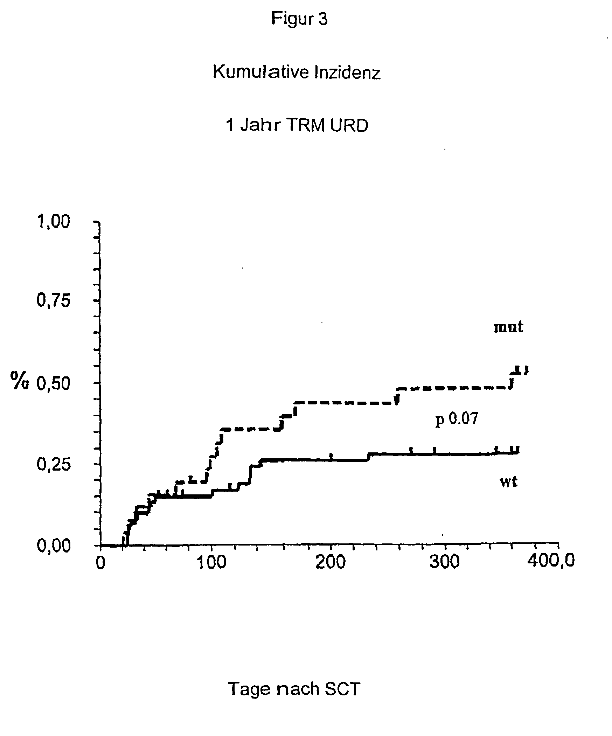

- Figur 2+3:: zeigt die kumulative Inzidenz von 1 Jahr TRM in Bezug auf NOD2/CARD15 SNPs: wt = nicht-mutante SNPs sowohl in Empfängern als auch in Spendern; mut = irgendein mutiertes SNP in Spendern oder Empfängern oder in beiden.
- Figur 2: zeigt die HLA-identischen Schwester-Transplantate; wt: n=55, mut n=23
- Figur 3: zeigt HLA nicht verwandte Spender-Transplantate; wt: n= 61, mut; n = 25

### Beispiele

### Methoden

169 aufeinander folgende Patienten, die für die allogenen Stammzelltransplantation aufgenommen wurden, wurden in die Studie einbezogen. Konditionierung und prophylaktische Immunsuppression wurde nach Standardprotokollen durchgeführt. Konditionierung mit reduzierter Intensität (RIC) bestand aus Fludarabine, BCNU und Melphalan. 78 Patienten erhielten Transplantate von HLA-identischen Geschwistern (MRD, matched related donors), 87 Patienten ohne Beziehung zu Spendern (URD, unrelated donors) und in 4 Patienten diente ein Verwandter, der sich in einem HLA-Antigen unterschied (RD) als Spender. In der nicht-verwandten Spendergruppe passten Spender/Empfänger Paare für HLA A, B, DRB1 und DQB1 wie durch Niedrigauflösung durchgeführte Typisierung für Klasse 1 und durch Hochauflösung durchgeführte Typisierung für Klasse II festgelegt wurden. Wohingegen in den verbleibenden 23 Paaren eine oder zwei Allelabweichungen für DRB1 und DQB1 akzeptiert wurden. Der Grad der GvHD wurde gemäß den Glucksberg-Kriterien (Glucksberg H et al. Transplantation 1974, 18, 295-304) bestimmt. Die Hauptvariablen wie Transplantat bezogene Mortalität (TRM= Tod in Remission) und die Todesursachen wurden in eine monatliche aktualisierte Datenbank aufgenommen. Mediane Beobachtungszeit für überlebende Patienten oder nach einem Rezidiv sterbende Patienten war 450 Tage (Bereich 30-1767 Tage), mediane Beobachtungszeit für an TRM sterbende Patienten war 172 Tage (Bereich 14-1065 Tage).

### Kollektion von DNA-Proben

DNA aus 169 Patienten (Empfänger) und von 168 Spendern wurde zum Zeitpunkt der Stammzelltransplantation aus Blutproben nach Standardmethoden präpariert, eingefroren und retrospektiv analysiert, um die Rolle von NOD2/CARD15 Mutationen zu beurteilen.

### Allellische Diskriminierung des NOD2/CARD15 Gens:

Einzelnukleotid Polymorphismen NOD2-SNP8, NOD2-SNP12 und NOD2-SNP13 wurden durch ein Taqmanprotokoll bestimmt wie in Hampe et al. The Lancet, 2002, 359, 1661-1665 beschrieben.

Kontrollproben, die durch DNA-Sequenzierung überprüft wurden, wurden in jedem TaqMan Lauf miteinbezogen. Sonden wurden mit einem Reporter Farbstoff 6-FAM oder TET kovalent am 5' Ende und der Quencher Farbstoff TAMRA wurde am 3' Ende der Sonde gekoppelt. Die Primer und Sonden wurden von MWG Biotech (Ebersberg, Deutschland) synthetisiert. Folgende Primer und Sonden wurden verwendet:

### Für NOD2-SNP8 Polymorphismus

- Primer:: 1) 5' TTCCTGGCAGGGCTGTTGTC 3' (vor)
2) 5' AGTGGAAGTGCTTGCGGAGG 3' (rück)
- Sonde:: 1) 5'FAM-CCTGCTC**C**GGCGCCAGGC-TAMRA 3'
2) 5' TET-CCTGCTC**T**GGCGCCAGGC- TAMRA 3'

### Für NOD2-SNP12 Polymorphismus

- Primer:: 1) 5' ACTCACTGACACTGTCTGTTGACTCT 3' (vor)
2) 5' AGCCACCTCAAGCTCTGGTG 3' (rück)
- Sonde:: 1) 5'FAM-TTTTCAGATTCTG**G**GGCAACAGAGTGGGT-TAMRA 3'
2) 5' TET- TTCAGATTCTGG**C**GCAACAGAGTGGGT - TAMRA 3'

### Für NOD2-SNP13 Polymorphismus

- Primer:: 1) 5' GTCCAATAACTGCATCACCTACCTAG 3' (vor)
2) 5' CTTACCAGACTTCCAGGATGGTGT 3' (rück)
- Sonde:: 1) 5' FAM-CCCTCCTGCAGG**CCC**TTGAAAT-TAMRA 3'
2) 5' TET- CCTCCTGCAGG**CCCC**TTGAAA - TAMRA 3'

Der optimierte Reaktionsmix mit einem Endvolumen von 10 µl bestand aus Universal Master Mix (PE Applied Biosystems), 400 nM von jedem Primer (vor und rück, siehe oben), 250 nM von jeder fluorogenen Sonde (siehe oben) und die DNA-Matrize (20 ng/well). Alle Reaktionen wurden in einer 384-Lochplatte (Abgene, epsom, Uk) durchgeführt und mit Hilfe des Thermocyclers Primus-HT (MWG Biotech) amplifiziert. Die Konditionen für die PCR-Zyklen waren wie folgt: 50°C für 2 Minuten, dann 95°C für 10 Minuten gefolgt von 40 Zyklen von 95°C für 15 Sekunden (Schmelzschritt) und 60°C für 1 Minute. Nach dem PCR-Lauf wurde die freigesetzte Fluoreszenz mit dem ABI PRISM^{®} 7700 Sequenz-Detektionssystem (PE Applied Biosystems, Forster City, CA, USA) gemessen.

Erhöhungen in der Menge an Reporterfarbstofffluoreszenz während der 40 Zyklen der Amplifikation wurden durch den Sequenz Detektor (SDS Version 1.6, PE Applied Biosystem) aufgezeichnet. Veränderungen in der Reporter Farbstofffluoreszenz des 6-FAM gegen Veränderung des Reporterfarbstoffs TET wurden graphisch ermittelt (homozygotes FAM gegen homozygotes TET gegen heterozygotes FAM/TET).

### Klinische und statistische Analysen

Drei Hauptvariablen (TRM an 365 Tagen = 1 Jahr TRM, Gesamt-Grad von schweren GvHD (Grad III/IV) und Stadium von gastrointestinalen GvHD) wurden in Bezug auf NOD2/CARD15 Mutationen analysiert. Kumulative Inzidenz, die das konkurrierende Risiko des Todes von anderen Toxizitäten oder Rezidiven von GvHD reflektiert und Todesfälle von Rezidiven für TRM wurden für jeden dieser Parameter berechnet. Die Zeit zum klinischen Ereignis (TRM/GvHD) wurde ab Stichtag des SCT (Stammzelltransplantation) für Gesamt-GvHD und gastrointestinales GvHD gemessen. Die klinischen Ereignisse wurden bis Tag 100 und für 1 Jahr TRM bis Tag 365 nach SCT in das Modell miteinbezogen. Schrittweise multivariante COX-Regressionsmodelle wurden angepasst und testeten die unabhängige prognostische Relevanz von NOD2/CARD15 Mutationen. Die Grenze für reverse Selektionsprozeduren war die Fehlerwahrscheinlichkeit 0.2. Für den multivarianten Vergleich von Risikofaktoren für TRM und GvHD wurde nur das Alter der Patienten, Krankheitsstadium am Tage der Transplantation, Spendertyp (HLA-identische Geschwister versus URD versus RD) und NOD2/CARD15 Mutationsstatus betrachtet. Für die Analyse von NOD2/CARD15 SNPs wurde die weniger häufigen mit Crohn's Krankheit und einer eingeschränkten NF-kB Produktion assoziierten Hochrisikoallele als mutiert definiert wohingegen die häufiger beobachteten Allele als Wildtyp definiert wurden. Für die Analyse der Mutationen in Bezug auf das klinische Ereignis wurden die Gruppen gemäß 1) Auftreten von irgendwelchen Mutationen in Empfänger und Spendern und 2) in Bezug auf das Auftreten von irgendwelchen Mutation in Empfängern allein, Spender allein oder beide gebildet. Kumulatives Auftreten erlaubt die Einstellung auf konkurrierendes Risiko, das mit Hilfe NCSS Software (Version 2004), weitere statistische Analysen durch die Hilfe von der SPSS Software (Version 11.05) berechnet wurde.

### Ergebnisse:

### Frequenz der mutierten NOD2/CARD15 Allele:

NOD2/CARD15 Mutationen ereigneten sich mit einer Häufigkeit von 21,8% in Patienten und 13,7% in Spendern, die in dieser Studie einbezogen wurden. Eine homozygote Mutation wurde in nur einem Patienten und in seinem HLA-identischen Spender beobachtet, wohingegen alle anderen Patienten und Spender mit Mutationen heterozygot waren. Berechnete Haplotyphäufigkeit für mutiertes NOD2-SNP8 war 0.056 für Empfänger (R) und 0.045 für Spender (D), für mutiertes NOD2-SNP12 0.021 (R) und 0.009 (D), und für mutiertes NOD2-SNP13 0.027 (R ) und 0.034 (D). Deshalb waren die Haplotyphäufigkeiten nahe dem Bereich der Kontrollen. Dies resultierte in einem Gesamtprozentsatz von 70,5% der Transplantate, wo sowohl Spender als auch Empfänger nicht-mutierte SNPs (Wildtypgruppe) hatten. In 22 Paaren (13%) hatten nur die Empfänger mutierte SNPs (R mut), in 16 Paaren (9,5%) traten Mutationen nur in Spendern auf (D mut), wohingegen in 12 Paaren (7%) sowohl Spender als auch Empfänger Mutationen offenbarten (R & D mut). In unserer aufeinander folgende Kohorte von Patienten waren sowohl die Spender spezifischen Charakteristiken als auch die Transplantat spezifische Prozeduren gleichmäßig zwischen Patienten/Spender Paaren mit und ohne Mutationen verteilt.

### Klinisches Ereignis in Bezug zum Auftreten von NOD2/CARD15 Mutationen in Spender und Empfängern

Patienten wurden für ein Median von 16 Monaten verfolgt (Bereich 0,5 bis 59 Monate). Zuerst verglichen wir das Ereignis bei Patienten von Wildtyp Empfänger/Spender Paar (n=119) mit dem Ereignis bei Patienten in Paaren mit irgendwelchen Mutationen (n=50): Im allgemeinen war schweres GvHD Grad III/IV, schweres gastrointestinales GvHD und TRM nach 1 Jahr in Paaren mit NOD2/CARD15 Mutationen verglichen mit der Wildtypgruppe signifikant erhöht (siehe Tabelle 1) Todesfälle von Rezidiven traten in beiden Gruppen auf: 18 in den 119 Patienten mit Wildtyp und 7 aus 50 Patienten in mutierten Paaren.
Es gab ein erhöhtes Risiko von schweren Gesamt-GvHD in Transplantaten mit Spendermutationen, wenn die Mutationen gemäß dem Auftreten in Empfänger oder Spender oder in beiden getrennt wurde. Zusätzlich gab es eine dramatische Erhöhung für die Gesamt- und gastrointestinale GvHD in den kleinen Subgruppen von Paaren mit Mutationen von Empfänger als auch Spendern. Wie in Tabelle 2 gezeigt, erhöhte sich das kumulative Auftreten von 1 Jahr TRM von 20% im Wildtyp-Empfänger /Spender Paare auf 49% in Paaren die eine Empfängermutation (p=00.3), auf 59% in Paaren, die eine Spendermutation (p=0.004) und auf 83% in Paaren, die eine Empfänger und Spendermutation tragen (p=0.001). Nochmals, Todesfälle von Rezidiven waren fast gleichmäßig in diesen Subgruppen verteilt.

Die starke Assoziation von TRM mit NOD2/CARD15 Mutation wurde partiell durch ein erhöhtes Risiko nicht nur für den Tod durch GvHD aber auch durch respiratorisches Versagen in Folge diffuser primärer oder sekundärer pulmonarer Schäden erklärt: Wohingegen in der Wildtypgruppe nur 11/30 Todesfällen (37%) in Remissionen durch GvHD und respiratorisches Versagen verursacht wurde. Der Anteil erhöhte sich zu 17/26 (65%) in Paaren mit NOD2/CARD15 Mutationen.

Figur 1 und Tabelle 2 zeigen die kumulative Inzidenz (Kum. Inz.) von schwerer akuten GvHD (Grad III/OV) in Bezug zu NOD2/CARD15 SNPs. Im Vergleich zu Widtyp (19%) erhöhte sich die kumulative Inzidenz auf 36% bzw. 44% im Falle von Mutationen entweder im Empfänger oder Spender und auf 58% im Falle von Mutationen in beiden (R & D mut).

### Relevanz der Beobachtungen für HLA-identische Geschwister gegenüber nicht-verwandten Spendertransplantaten und multivariante Analyse des Risikofaktors assoziiert mit TRM;

Die Relevanz von Spendern und Empfänger-Mutationen wurden in den verschiedenen immunogenen in dieser Studie einbezogenen Subgruppen verglichen. Obwohl die Korrelation des Auftretens von TRM mit NOD2/CARD15 Mutationen deutlicher für die HLA-identischen Geschwistertransplantate war, gab es einen parallelen Trend für URD Transplantate (siehe Figur 2, Tabelle 3 ). In der URD Gruppe stieg das kumulative Auftreten der 1 Jahr TRM von 27% in Wildtyppaaren auf 55% in mutanten Paaren, wenn der Empfänger und Spender für HLA A, B, DRB1 und DQB1 glichen. TRM erhöhte sich von 29% auf 50 % in Paaren mit ein oder zwei DRB1 oder DQB1 Allel-Abweichungen.

Die kleine Gruppe von Transplantaten, die Antigenabweichungen aufzeigen, zeigte eine hohe Mortalität und erlaubte keine Beurteilung der Rolle von NOD2/CARD15 Mutationen in dieser besonderen Subgruppe. In der multivarianten Cox Regressionsanalyse waren Alter, Spendertyp und NOD2/CARD15 Mutationen in Spendern alleine oder in Empfänger als auch in Spender unabhängige Risikofaktoren für TRM. Wie in Tabelle 4 gezeigt war das Zufallsverhältnis (hazard ratio) für TRM nach allogener SCT betreffend NOD2/CARD15 Mutation 2.4 (p= 0.02; 95% Cl [1.1;5.0]) für Empfänger-Mutationen, 2.5 (p = 0.02; 95% Cl [1.2; 5.4]) für Spender-Mutationen und 6.0 (p=0; 95% Cl [2.6;14.1]) im Fall der simultanen Mutationen von Spender und Empfänger. GvHD (p = 0.006) als auch NOD2/CARD15 Mutationen (p= 0) blieben signifikante Risikofaktoren für TRM, wenn die allgemeine GvHD Grad IV als Risikofaktor für TRM in das Modell einbezogen wurde. Sogar wenn schwere GvHD Grad III/IV einbezogen wurde, blieb die Verteilung der NOD2/CARD15 Mutation signifikant, dass die starke und unabhängige Auswirkung der NOD2/CARD15 Mutation auf den Krankheitsverlauf (Befund) nach allogener SCT bestätigte.

**Tabelle 1:**

| | Kumulative Inzidenz | 95% Kl Kum.Inz. | p | HR | 95% Kl HR |
|---|---|---|---|---|---|
| GvHD III/IV | | | | | |
| Wildtyp | 19% | 13% - 27% | | 1,0 | |
| Mutante | 44% | 32% - 60% | 0,001 | 2,7 | 1,5 - 4,9 |
| GI GvHD | | | | | |
| Wildtyp | 18% | 12% - 27% | | 1,0 | |
| Mutante | 40% | 28% - 56% | 0,004 | 2,5 | 1,4 - 4,7 |
| TRM 1 Jahr | | | | | |
| Wildtyp | 20% | 14% - 29% | | 1,0 | |
| Mutante | 58% | 45% - 75% | 0 | 2,8 | 1,7 - 4,9 |

- Tabelle 1: zeigt die univariante Analyse der kumulativen Inzidenz der aktuen GvHD Grad III/IV, gastrointestinales GvHD Stadium 2-4 und 1 Jahr TRM. Patienten/Spender-Paare mit nicht-mutiertem (Wildtype) NOD2/CARD15 (n=119) wurden gegenüber den Paaren mit Empfänger- oder Spender-Mutationen (mutated, n=50) verglichen. Cox-Regression wurde eingesetzt um die Gruppen zu vergleichen und Hazard Ratios zu analysieren.

**Tabelle 2:**

| | Kumulative Inzidenz | 95% Kl Kum. Inz. | p | HR | 95% Kl HR |
|---|---|---|---|---|---|
| GvHD III/IV | | | | | |
| Wildtyp | 19% | 13% - 27% | | 1,0 | |
| R pos | 36% | 21% - 61% | 0,07 | 2,1 | 0,9 - 4,7 |
| D pos | 44% | 25% - 76% | 0,002 | 2,8 | 1,2 - 6,5 |
| R & D pos | 58% | 36% - 94% | 0,002 | 3,9 | 1,7 - 9,2 |
| GI GvHD | | | | | |
| Wildtyp | 18% | 12% - 27% | | 1,0 | |
| R pos | 36% | 21% - 63% | 0,07 | 2,1 | 0,9 - 4,8 |
| D pos | 31% | 15% - 65% | 0,16 | 2,0 | 0,8 - 5,3 |
| R & D pos | 56% | 36% - 94% | 0,001 | 4,3 | 1,8 - 10,1 |
| TRM 1 Jahr | | | | | |
| Wildtyp | 20% | 14% - 29% | | 1,0 | |
| R pos | 49% | 31% - 76% | 0,03 | 2,2 | 1,1 - 4,6 |
| D pos | 59% | 38% - 91% | 0,004 | 3,1 | 1,4 - 6,5 |
| R & D pos | 83% | 65% - 100% | 0,001 | 3,9 | 1,7 - 8,6 |

- Tabelle 2: zeigt die detaillierte kumulative Inzidenz für GvHD Grad III/IV, gastrointestinales Stadium 2-4 und 1 Jahr TRM bezogen auf Spender und Empfänger NOD2/CARD15 Mutationen: Wildtype: nicht-mutierte Spender und Empfänger (n=119). R pos = Mutation nur in Empfängern (n=22), D pos = Mutationen nur in Spendern (n=16); R & D pos = Mutationen sowohl in Empfängern als auch in Spendern (n = 12). Cox-Regression wurde eingesetzt um die Gruppen zu vergleichen und Hazard Ratios zu analysieren.
- Tabelle 3: zeigt die univariante Analyse der kumulativen Inzidenz von 1 Jahr TRM in Patienten/Spender Paaren mit Wildtyp NOD2/CARD15 gegenüber Paaren mit entweder Empänger- (R pos) oder Spender-Mutationen (D pos) sowie Mutationen in beiden (R & D pos). Separate Analysen wurden für HLA-identische Geschwister Transplantation und URD Transplantate durchgeführt. Der Vergleich zwischen den Gruppen wurde mit der log rank Analyse durchgeführt. 95 Cl confidence intervals.

**Tabelle 3:**

| | Kumulative Inzidenz | 95% Kl Kum. Inz. | p | HR | 95% Kl HR |
|---|---|---|---|---|---|
| HLA-id. sibl. | | | | | |
| Wildtyp (55) | 14% | 18% - 42% | | 1,0 | |
| R pos (9) | 74% | 49% - 100% | 0, | 7,9 | 2,6 - 24,0 |
| D pos (6) | 58% | 27% - 100% | 0,022 | 4,9 | 1,3 - 18,9 |
| R & D pos (8) | 75% | 50% - 100% | 0,004 | 6,4 | 1,8 - 23,0 |
| URD | | | | | |
| Wildtyp (61) | 28% | 18% - 42% | | 1,0 | |
| R pos (13) | 36% | 14% - 73% | | 1,1 | 0,4 - 3,3 |
| D pos (9) | 31% | 13% - 100% | | 2,4 | 0,9 - 6,5 |
| R & D pos (4) | 56% | 100% | 0,01 | 4,2 | 1,4 - 12,5 |

- Tabelle 4: zeigt die multivariante Risikofaktor-Analyse für 1 Jahr TRM. Relevante Patienten und Spender Prätransplantatcharakteristika wurden mit NOD2/CARD15 Mutation in Empfängern (R pos), Spendern (D pos) und beiden (R & D ps) verglichen.

**Tabelle 4:**

| | N | Auftritt TRM 1Jahr | Risiko | 95% Cl | p |
|---|---|---|---|---|---|
| Alter | | | | | |
| bis 40 Jahre | 58 | 15 | 1,0 | | |
| ab 40 Jahre | 111 | 38 | 2,2 | 1,1 - 4,0 | 0,02 |
| Stadium bei Tx | | | | | |
| früh | 95 | 27 | 1,0 | | |
| fortgeschritten | 74 | 26 | 1,3 | 0,7 - 2,2 | |
| Donortyp | | | | | |
| HLA = sibl | 78 | 20 | 1,0 | | |
| nicht verw. | 87 | 29 | 1,7 | 0,9 - 3,2 | |
| HLA df.rel. | 4 | 4 | 10,7 | 3,4 - 33,3 | 0 |
| NOD2/CARD15 | | | | | |
| Wildtyp | 119 | 26 | 1,0 | | |
| R pos | 22 | 10 | 2,4 | 1,1 - 5,0 | 0,02 |
| D pos | 16 | 9 | 2,5 | 1,2 - 5,4 | 0,02 |
| R & D pos | 12 | 8 | 6,0 | 2,6 - 14,1 | 0,001 |

## Patentansprüche

1. Verfahren zur Vorhersage und/oder Diagnose von mit mindestens einem der Polymorphism SS 2978536, Allel T(Nod2-SNP8), SS 2978537, Allel C (Nod2-SNP12), SS 2978539, Allel Insution C (Nod2-SNP13) im NOD2/CARD15 Gen assoziierten Krankheiten durch Nachweis mindestens eines der Polymorphismen Nod2-SNP8, Nod2-SNP12, Nod2-SNP13 im NOD2/CARD15 Gen, wobei es sich bei den mit den Polymorphismen Nod2-SNP8, Nod2-SNP12, Nod2-SNP13 im NOD2/CARD15 Gen assoziierten Krankheiten um Abstoßungsreaktionen bei Transplantationen, Graft-versus-Host-Erkrankungen, Host-versus-Graft-Erkrankungen und Bronchiolitis obliterans handelt.

2. Verfahren nach Anspruch 1, umfassend die Schritte
a) Bereitstellung einer Probe enthaltend das Gen NOD2/CARD15 bzw. NOD2/CARD15-Nukleinsäuren,
b) Untersuchung des Gens NOD2/CARD15 auf das Vorliegen mindestens eines der Polymorphismen Nod2-SNP8, Nod2-SNP12, Nod2-SNP13.

3. Verfahren nach einem der Ansprüche 1 oder 2, umfassend die Schritte
a) Bereitstellung einer Probe enthaltend das Gen NOD2/CARD15,
b) Isolation der DNA und/oder RNA aus der Probe,
c) Durchführung einer PCR mit für das Gen NOD2/CARD15 spezifischen Primern,
d) Untersuchung des Gens NOD2/CARD15 auf das Vorliegen mindestens eines der Polymorphismen Nod2-SNP8, Nod2-SNP12, Nod2-SNP13.

4. Verfahren zur Vorhersage der Wahrscheinlichkeit des Auftretens von Abstoßungsreaktionen bei Transplantationen gemäß eines der vorherigen Ansprüche, umfassend die folgenden Schritte:
a) Bereitstellung einer Probe des Spenders enthaltend das Gen NOD2/CARD15 sowie eine Probe des Empfängers enthaltend das Gen NOD2/CARD15,
b) Detektion der beiden Proben auf das Vorliegen eines oder mehrerer der Polymorphismen Nod2-SNP8, Nod2-SNP12, Nod2-SNP13.

5. Verfahren nach einem der Ansprüche 1 **-** 4, wobei bei dem Verfahren mindestens ein Oligonucleotid bestehend aus mindestens 10 Nucleotiden verwendet wird, wobei das Oligonucleotid eine zum NOD2/CARD15 Gen komplementäre Sequenz hat und das komplementäre Nucleotid zur Mutation SNP8 und/oder SNP12 und/oder die Nucleotidinsertion SNP13 enthält.

6. Verfahren nach Anspruch 5, wobei das Oligonucleotid des weiteren einen Detektionsmarker enthält.

7. Verfahren nach einem der Ansprüche 1 - 6, wobei das Verfahren unter Verwendung von mindestens einem Mikrochip oder Diagnosechip durchgeführt wird, wobei der Mikrochip oder Diagnosechip mindestens ein Oligonucleotid bestehend aus mindestens 10 Nucleotiden enthält, wobei das Oligonucleotid eine zum NOD2/CARD15 Gen komplementäre Sequenz hat und das komplementäre Nucleotid zur Mutation SNP8 und/oder SNP12 und/oder die Nucleotidinsertion SNP13 enthält.

8. Verfahren nach Anspruch 7, wobei das Oligonucleotid des weiteren einen Detektionsmarker enthält.

## Claims

1. A method for prediction and/or diagnosis of diseases associated with at least one of the polymorphisms ss2978536, allele T, ss2978537, allele C, ss2978539, allele insertion C in the NOD2/CARD15 gene by proving at least one of the polymorphisms Nod2-SNP8, Nod2-SNP12, Nod2-SNP13 in the NOD2/CARD15 gene, wherein the diseases associated with at least one of the polymorphisms Nod2-SNP8, Nod2-SNP12, Nod2-SNP13 in the NOD2/CARD15 gene are rejection reactions in transplantations, graft-versus-host diseases, host-versus-graft diseases and bronchiolitis obliterans.

2. The method according to claim 1 comprising the steps
a) Providing a sample comprising the gene NOD2/CARD15, respectively NOD2/CARD15 nucleic acids,
b) Screening the gene NOD2/CARD15 for the presence of at least one of the polymorphisms Nod2-SNP8, Nod2-SNP12, Nod2-SNP13.

3. The method according to any of claims 1 or 2 comprising the steps
a) Providing a sample comprising the gene NOD2/CARD15,
b) Isolating the DNA and/or RNA from the sample,
c) Performing a PCR with primers specific for the gene NOD2/CARD15,
d) Screening the gene NOD2/CARD15 for the presence of at least one of the polymorphisms Nod2-SNP8, Nod2-SNP12, Nod2-SNP13.

4. A method for predicting the probability for the occurrence of rejection reactions in transplantations according to any of the previous claims, comprising the following steps:
a) Providing a sample of the donor containing the gene NOD2/CARD15 and a sample of the host containing the gene NOD2/CARD15,
b) Screening both samples for the presence of one or more of the polymorphisms Nod2-SNP8, Nod2-SNP12, Nod2-SNP13.

5. The method according to any of claims 1 to 4, wherein at least one oligonucleotide consisting of at least 10 nucleotides is used in the method, wherein the oligonucleotide has a sequence complementary to the gene NOD2/CARD15 and contains the nucleotide complementary to the mutation SNP8 and/or SNP12 and/or to the nucleotide insertion SNP13.

6. The method according to claim 5, wherein the oligonucleotide further comprises a detection marker.

7. The method according to any of claims 1 to 6, wherein the method is carried out using at least one microchip or diagnostic chip, wherein the microchip or diagnostic chip contains at least one oligonucleotide consisting of at least 10 nucleotides, wherein the oligonucleotide has a sequence complementary to the gene NOD2/CARD15 and contains the nucleotide complementary to the mutation SNP8 and/or SNP12 and/or to the nucleotide insertion SNP13.

8. The method according to claim 7, wherein the oligonucleotide further comprises a detection marker.

## Revendications

1. Un procédé pour le pronostic et/ou diagnose de maladies associées avec au moins un des polymorphismes ss2978536, allèle T, ss2978537, allèle C, ss2978539, allèle insertion C dans le gène NOD2/CARD15 par la preuve d'au moins un des polymorphismes Nod2-SNP8, Nod2-SNP12, Nod2-SNP13 dans le gène NOD2/CARD15, dans lequel les maladies associées avec au moins un des polymorphismes Nod2-SNP8, Nod2-SNP12, Nod2-SNP13 dans le gène NOD2/CARD15 sont des réactions de rejet chez une transplantation, des maladies du greffon contre l'hôte, des maladies de l'hôte contre le greffon et de la bronchiolite oblitérante.

2. Le procédé selon la revendication 1 comprenant les étapes consistant à
a) Fournir un prélèvement comprenant le gène NOD2/CARD15, respectivement les acides nucléiques NOD2/CARD15,
b) Examiner le gène NOD2/CARD15 sur la présence d'au moins un des polymorphismes Nod2-SNP8, Nod2-SNP12, Nod2-SNP13.

3. Le procédé selon une des revendications 1 ou 2 comprenant les étapes consistant à
a) Fournir un prélèvement comprenant le gène NOD2/CARD15,
b) Isoler l'ADN et/ou l'ARN de ce prélèvement,
c) Accomplir une PCR avec des amorces spécifiques pour le gène NOD2/CARD15,
d) Examiner le gène NOD2/CARD15 sur la présence d'au moins un des polymorphismes Nod2-SNP8, Nod2-SNP12, Nod2-SNP13.

4. Un procédé pour pronostiquer la probabilité de la survenance d'une réaction de rejet chez une transplantation selon une des revendications antérieures, comprenant les étapes suivantes consistant à
a) Fournir un prélèvement du donneur contenant le gène NOD2/CARD15 et un prélèvement de l'hôte contenant le gène NOD2/CARD15,
b) Examiner les deux prélèvements sur la présence d'un ou plus des polymorphismes Nod2-SNP8, Nod2-SNP12, Nod2-SNP13.

5. Le procédé selon une des revendications 1 à 4, dans lequel au moins un oligonucléotide consistant d'au moins 10 nucléotides est utilisé dans le procédé, dans lequel l'oligonucléotide a une séquence complémentaire au gène NOD2/CARD15 et contient le nucléotide complémentaire à la mutation SNP8 et/ou SNP12 et/ou à l'insertion de nucléotide SNP13.

6. Le procédé selon la revendication 5, dans lequel l'oligonucléotide comprend en outre un marqueur de détection.

7. Le procédé selon une des revendications 1 à 6, dans lequel le procédé est réalisé en utilisant au moins un microchip ou un chip de diagnostic, dans lequel le microchip ou le chip de diagnostic contient au moins un oligonucléotide consistant d'au moins 10 nucléotides, dans lequel l'oligonucléotide a une séquence complémentaire au gène NOD2/CARD15 et contient le nucléotide complémentaire à la mutation SNP8 et/ou SNP12 et/ou à l'insertion de nucléotide SNP13.

8. Le procédé selon la revendication 7, dans lequel l'oligonucléotide comprend en outre un marqueur de détection.
